# EUROPEAN PATENT APPLICATION

(11) **EP 2 433 567 A1**
(43) Date of publication of application: **28.03.2012**
(21) Application number: 10777745.0
(22) Date of filing: 18.05.2010
(51) Int. Cl.: A61B 8/08

(54) **MEDICAL IMAGE DIAGNOSIS DEVICE AND REGION-OF-INTEREST SETTING METHOD THEREFOR**

(30) Priority: 20.05.2009 JP 2009121508
(71) Applicant: Hitachi Medical Corporation, Chiyoda-ku Tokyo 101-0021 (JP)
(72) Inventor: CHONO,Tomoaki, Tokyo 101-0021 (JP)
(74) Representative: MERH-IP Matias Erny Reichl Hoffmann
(86) International application number: PCT/JP2010/058335
(87) International publication number: WO 2010/134512

(57) **Abstract**

A medical image diagnosis device according to the present invention has a medical image acquiring unit that acquires a medical image, a three-dimensional image constructing unit that constructs a three-dimensional image containing a motional internal organ region in the medical image, a sectional image generator that generates a two-dimensional sectional image serving as a reference image from the three-dimensional image, a region dividing unit that divides the reference image into plural regions on the basis of a criterion for region division, and a region-of-interest setting unit that calculates motion states of the plural regions, specifies at least one region of the plural regions on the basis of the calculated motion states, and sets as a region of interest a region of the medical image in which the specified region is contained.

## Description

### Technical Field

The present invention relates to a medical image diagnosis device that can enhance operability for setting of a region-of-interest (ROI) in a medical image, and a region-of-interest setting method therefor.

### Background Art

A medical image diagnosis device is used by an examiner such as a medical doctor, a clinical laboratory technician or the like. The examiner sets ROI in a diagnosis region as a part of an image when using the medical image diagnosis device.

The examiner sets ROI by tracing a partial region of an image displayed on a display screen with a pointing device. The tracing operation executed with an examiner's hand is called as an ROI manual setting. When it is necessary to set ROI in each of plural images acquired by the medical image diagnosis device, the ROI manual setting becomes a cumbersome operation for the examiner.

Furthermore, it has been known that ROI set for a motional internal organ such as a heart or the like has regularity in size, displacement, etc. of ROI based on a motion of a heart or the like. Therefore, it is possible to set ROI in a motional internal organ by creating an image processing program for a computer according to the above regularity and executing the image processing program. In the description of this patent application, it is referred to as "ROI automatic setting" that ROI is extracted by the image processing program of the image processing.

An ROI automatic setting method has been proposed in Patent Document 1, for example. In Patent Document 1, a computer (CPU) is made to extract the boundary between a cardiac cavity and a cardiac muscle on the basis of gradient of density on an ultrasonic image by using an automatic contour tracking (ACT) method and treat as ROI a region which is limited by the boundary.

### Prior Art

### Patent Document

Patent Document 1: JP-A-11-155862

### Summary of the Invention

### Problem to be solved by the Invention

However, in Patent Document 1, an image to be treated is a two-dimensional image, and ROI automatic setting for a three-dimensional image is not suggested.

Therefore, an object of the present invention is to provide a medical image diagnosis device that can perform ROI automatic setting for a three-dimensional image of a motional internal organ, and a region-of-interest setting method therefor.

### Means of solving the Problem

In order to solve the above problem, according to the present invention, a reference sectional image is generated from a three-dimensional image of a motional internal organ, the generated reference sectional image is divided into plural regions on the basis of a criterion for region division, a region having a different motion state is specified from the plural divided regions, and a region of interest is set in a region on a medical image which contains the specified region.

Specifically, a medical image diagnosis device according to the present invention is characterized by including: a medical image acquiring unit that acquires a medical image; a three-dimensional image constructing unit that constructs a three-dimensional image containing a motional internal organ region in the medical image; a sectional image generator that generates a two-dimensional sectional image serving as a reference image from the three-dimensional image; a region dividing unit that divides the reference image into plural regions on the basis of a criterion for region division; and a region-of-interest setting unit that calculates motion states of the plural regions, specifies at least one region of the plural regions on the basis of the calculated motion states, and sets as a region of interest a region of the medical image in which the specified region is contained.

According to the medical image diagnosis device of the present invention, a medical image is acquired by the medical image acquiring unit, a three-dimensional image containing a motional internal organ region in the medical image is constructed by the three-dimensional image constructing unit, a two-dimensional sectional image serving as a reference image is generated from the three-dimensional image by the sectional image generator, the reference image is divided into plural regions on the basis of a criterion for region division by the region dividing unit, the motion states of the plural regions are calculated by the region-of-interest setting unit, at least one region of the plural regions is specified on the basis of the calculated motion states, and the region of the medical image which contains the specified region is set as a region of interest, whereby the three-dimensional image of the motional internal organ can be divided into plural regions on the basis of a predetermined region dividing criterion, the motion states of the plural regions can be calculated every region, and a region whose motion state is different from motion states of the other regions can be set as ROI.

A region-of-interest setting method according to the present invention is characterized by including: a first step of acquiring a medical image by a medical image acquiring unit; a second step of constructing a three-dimensional image containing a motional internal organ region in the medical image by a three-dimensional image constructing unit; a third step of generating a two-dimensional sectional image serving as a reference image from the three-dimensional image by a sectional image generator; a fourth step of dividing the reference image into plural regions on the basis of a criterion for region division by a region diving unit; and a fifth step of calculating motion states of the plural regions, specifying at least one region out of the plural regions on the basis of the calculated motion states and setting as a region of interest a region of the medical image containing the specified region by a region-of-interest setting unit.

According to the region-of-interest setting method of the present invention, the first step acquires a medical image by the medical image acquiring unit, the second step constructs a three-dimensional image containing a motional internal organ in the medical image by the three-dimensional image constructing unit, the third step generates a two-dimensional sectional image serving as a reference image from the three-dimensional image by the sectional image generator, the fourth step divides the reference image into plural regions on the basis of a criterion for region division by the region dividing unit, and the fifth step calculates the motion states of the plural regions, specifies at least one region of the plural regions on the basis of the calculated motion states, and sets a region of the medical image containing the specified region as a region of interest by the region-of-interest setting unit, whereby the three-dimensional image of the motional internal organ can be divided into the plural regions on the basis of the predetermined region dividing criterion, the motion states of the plural regions can be calculated every region, and a region whose motion state is different from the motion states of the other regions can be set as ROI.

### Effect of the Invention

The present invention has an effect of proving a medical image diagnosis device and a region-of-interest setting method therefor in which a three-dimensional image of a motional internal organ is divided into plural regions on the basis of a predetermined region dividing criterion, the motion states of the plural regions are calculated every region, and a region whose motion state is different from the motion states of the other regions can be set as ROI.

### Brief Description of the Drawings

[Fig. 1] Fig. 1 shows an example of a system construction diagram of an ultrasonic image diagnosis device according to the embodiment 1 of the present invention.
[Fig. 2] Fig. 2 is a flowchart showing measurement processing of the ultrasonic image diagnosis device according to the embodiment 1 of the present invention.
[Fig. 3] Fig. 3 is a diagram showing an example of setting of a contour line of Fig. 2.
[Fig. 4] Fig. 4 shows a display example of the measurement processing of the ultrasonic image diagnosis device according to the embodiment 1 of the present invention.
[Fig. 5] Fig. 5 is a flowchart showing measurement processing of an ultrasonic image diagnosis device according to the embodiment 2 of the present invention.
[Fig. 6] Fig. 6 is a diagram showing an example of setting of a contour line of Fig. 5.
[Fig. 7] Fig. 7 is a diagram showing an example of setting of a contour line according to the embodiment 3 of the present invention.
[Fig. 8] Fig. 8 shows a display example of measurement processing of an ultrasonic image diagnosis device according to the embodiment 4 of the present invention.
[Fig. 9] Fig. 9 shows a display example of measurement processing of an ultrasonic image diagnosis device according to the embodiment 5 of the present invention.

### Modes for carrying out the Invention

Embodiments of the present invention will be described in detail with reference to the drawings.
An ultrasonic diagnosis device, an X-ray CT device, an MRI device, etc. are known as medical image diagnosis devices according to the present invention. In the embodiments of the present invention, an ultrasonic diagnosis device out of the medical image diagnosis devices will be exemplified.

### Embodiment 1

There will be described a case where a three-dimensional image containing a motional internal organ of an examinee is acquired by the ultrasonic diagnosis device, a two-dimensional reference image is extracted from the acquired three-dimensional image by CPU installed in the ultrasonic diagnosis device, and ROI is automatically set on the basis of the extracted two-dimensional reference image by CPU installed in the ultrasonic diagnosis device in the embodiment 1.

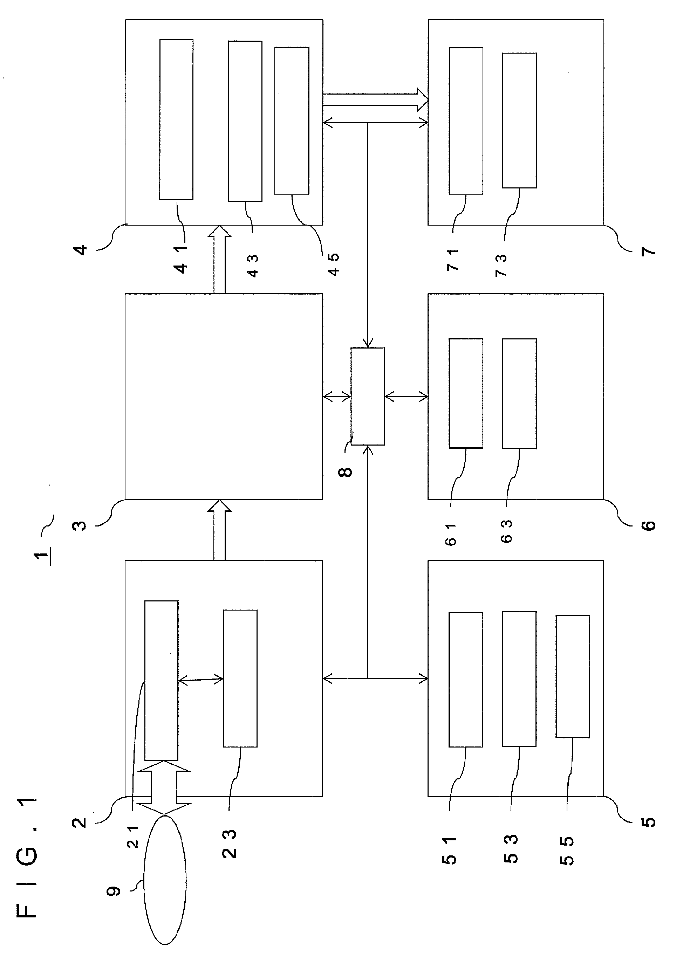

Fig. 1 is a block diagram schematically showing the ultrasonic diagnosis device according to the embodiment.
As shown in Fig. 1, the ultrasonic diagnosis device 1 has an ultrasonic signal generator 2, an ultrasonic image generator 3, an operating unit 4, a storage unit 5, a setting unit 6, a display unit 7 and a controller 8. In Fig. 1, solid-line arrows represent control, and outline arrows represent flow of image signal data.

The ultrasonic signal generator 2 has an ultrasonic probe 21 and an ultrasonic signal transmitting/receiving unit 23.
The ultrasonic probe 21 transmits ultrasonic waves to an examinee 9, and receives a reception signal from the examinee 9. The ultrasonic signal transmitting/receiving unit 23 passes the reception signal received by the ultrasonic probe 21 through a phasing addition circuit (not shown) to acquire a three-dimensional ultrasonic signal.

The ultrasonic probe 21 is classified in type in accordance with the arrangement direction of plural transducers. Specifically, the ultrasonic probe 21 is classified into a two-dimensional ultrasonic probe in which plural transducer elements are two-dimensionally arranged and a one-dimensional ultrasonic probe in which plural transducers are one-dimensionally arranged.

The two-dimensional ultrasonic probe can transmit/receive ultrasonic waves to/from the three-dimensional space, and it is suitable as the ultrasonic probe adopted in this invention because a three-dimensional ultrasonic signal is directly acquired.

Furthermore, a two-dimensional ultrasonic signal of an examinee can be acquired by the one-dimensional ultrasonic probe. According to a method of acquiring a three-dimensional ultrasonic signal by the one-dimensional ultrasonic probe, two-dimensional ultrasonic signals of the examinee are successively acquired in an orthogonal direction orthogonal to the arrangement direction of the transducers and stored in the storage unit 5 of the ultrasonic signal transmitting/receiving unit 23, and the phasing addition circuit executes an operation of arranging in the orthogonal direction the two-dimensional ultrasonic signals of the examinee which are successively acquired in the orthogonal direction, thereby constructing a three-dimensional ultrasonic signal.

The ultrasonic image generator 3 generates a three-dimensional image constructed by voxel data from the three-dimensional ultrasonic signal input from the ultrasonic signal generator 2 on the basis of a condition set in the setting unit 6.

The operating unit 4 has a two-dimensional section extracting unit 41, a two-dimensional contour line extracting unit 43 and an ROI/measurement value calculator 45.

The two-dimensional section extracting unit 41 executes an operation of extracting signals of specific sections from the three-dimensional ultrasonic signal. The specific sections are an image of a cardiac apex 2 cavity (A2C) and an image of a cardiac apex 4 cavity (A4C) which are reference images obtained by an echocardiogram examination. The image of A2C and the image of A4C are in such positional relationship as to be orthogonal to each other. The classification of each image is performed by a publicly known image recognition technique such as a block matching method or the like. Specifically, templates of A2C and A4C stored in the data base and the three-dimensional ultrasonic signal are subjected to comparison operation, and two-dimensional images formed by a three-dimensional ultrasonic signal having highest similarity through the comparison result are set as the image of A2C and the image of A4C.

The two-dimensional contour line extracting unit 43 extracts endocardiac and epicardiac contour lines of a heart on A2C and A4C.
In this description, a fractionating method recommended by American Society Echo-cardiography (ASE) (called as "ASE fractionating method") is used as a predetermined region dividing standard, for example. A region in which cardiac muscle is divided is called as a myocardial fraction.

The ROI/measurement value calculator 45 measures the size and motion of ROI, the motional internal organ by using the extracted endocardiac and epicardiac contour lines and the myocardial fraction. The myocardial fraction is acquired by the ASE fractionating method, for example.

In the following description, the ASE fractionating method is exemplified as a local region dividing method. However, as the local region dividing method may be adopted a publicly known region dividing method such as a labeling method of labeling (affixing numbers to) respective pixels and dividing (extracting) a region in which pixels are linked to one another, a K averaging method of classifying into K parts of the cluster number which is given by using an average of clusters or the like.

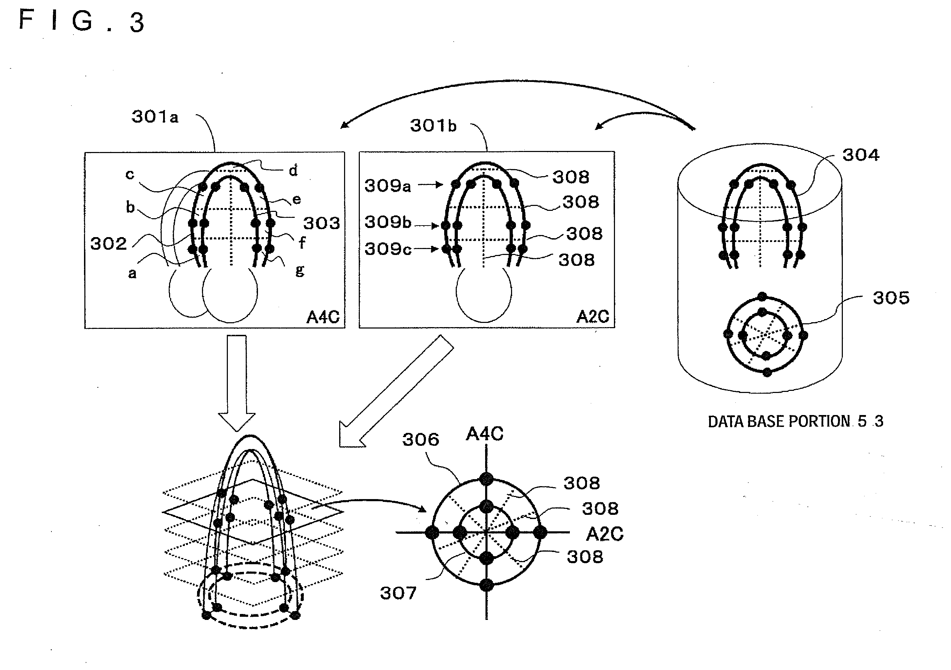

The region division based on the ASE fractionating method is performed by a contour line/fraction position setting unit 63 of the setting unit 6 described later. The contour line/fraction position setting unit 63 traces an internal organ region drawn on an ultrasonic image displayed on the display unit 7. In this case, a heart is set as an example of the internal organ. The contour line/fraction position setting unit 63 executes an operation of tracing the position on the endocardiac and epicardial images of the region in which the heart is drawn. The position information of the endocardium and the epicardium are represented by dual heavy lines as shown in sectional images 301a and 301b of Fig. 3. An outside heavy line 302 represents the epicardium, and an inner heavy line 303 represents the endocardia. The position information of the endocardium and the epicardium means the position at which the inner cavity portion and the cardiac muscle portion of the heart as a volume measurement target of the heart are separated from each other. In this case, there are three operations of manual operation, semi-automatic operation and automatic operation as an operation method executed by the examiner when the region of the drawn internal organ is traced.

(1) The manual operation is an operation method in which the examiner manually traces all the position information of the endocardium and the epicardium by using a pointing device. Specifically, the examiner traces the boundary between the regions corresponding to the endocardium and the epicardium while checking the image of the heart region on the ultrasonic image displayed on the display unit 7, thereby inputting the position information of the endocardium and the epicardium. The controller 8 temporarily stores the input position information of the endocardium and the epicardium into the storage unit 5.

(2) The semi-automatic operation is an operation method in which the examiner inputs plural points on the boundary of the region of the endocardium or the epicardium by using the pointing device and CPU extracts the boundary of the region of the endocardium or the epicardium on the basis of the plural points on the input boundary. Specifically, the examiner inputs plural boundary points between the region corresponding to the endocardium and the epicardium and a region adjacent to the corresponding region while checking the image of the heart region of the ultrasonic image displayed on the display unit 7. Upon receiving the input plural boundary points, the controller 8 connects the boundary points and makes the operating unit 4 execute interpolation calculation such as spline interpolation or the like so that the boundary lines of the regions are acquired as the position information of the endocardium and the epicardium. The controller 8 temporarily stores the input position information of the endocardium and the epicardium into the storage unit 5.

(3) The automatic operation is an operation method in which the examiner inputs pixel points in the region of the endocardium or the epicardium by using the pointing device and CPU extracts the boundary of the region of the endocardium or the epicardium on the basis of the input pixel points. Specifically, the examiner inputs one point to specify the regions corresponding to the endocardium and the epicardium while checking the image of the heart region of the ultrasonic image displayed on the display unit 7. The input one point serves as a seed in a region growing method. The controller 8 makes the operating unit 4 execute the region extracting operation based on the region growing method on the basis of the seed to acquire the boundaries of the regions as the position information of the endocardium and the epicardium. The controller 8 temporarily stores the acquired position information of the endocardium and the epicardium into the storage unit 5.

A predetermined dividing index is based on an ASE 16 fractionating method or 17 fractionating method of a cardiac muscle, for example. The 17 fractionating method, etc. are being regarded as industry standards in the measurement of the cardiac muscle which is performed by the medical image diagnosis device. When the 17 fractionating method is applied to the cardiac muscle, the examiner directly sets the positions of the 17 fractions to the cardiac muscle on the screen while checking the image on a image display unit 71, thereby inputting the positions of the fractions.

The storage unit 5 has a program storage unit 51, a data base portion 53 and an ROI/measurement value storing unit 55. A specific hardware of the storage unit 5 is a storage medium such as a semiconductor memory, a hard disk, an optical disk or the like.

In the program storage unit 51 are stored programs describing algorithms for the contour extraction processing, the measurement operation, etc. in the operating unit 4 and programs for controlling the respective parts.

The data base portion 53 stores local position information of the heart which contains position information of the two-dimensional section and the fraction position information of the cardiac muscle fraction, and contour data of the a two-dimensional contour shape when counter extraction using a counter model is applied.

The ROI/measurement value storing unit 55 stores a measurement value calculated by the ROI/measurement value calculator 45.

The setting unit 6 has a measurement condition setting unit 61 and the contour line/fraction position setting unit 63. The setting unit 6 is a user interface, and the specific hardware thereof is information input equipment containing a keyboard, a trackball and a switch.

The measurement condition setting unit 61 is used when the examiner manually sets parameters, and the set parameters are transmitted to the controller 8.

In addition to the function described above, when a contour or a fraction position extracted from the two-dimensional image is not set precisely, the contour line/fraction position setting unit 63 manually minutely adjusts the position concerned.

The display unit 7 has the image display unit 71 and an ROI/measurement value display unit 73. The hardware of the display unit 7 is a display device such as a CRT display, a liquid crystal display, a plasma display, an organic EL display or the like.

The image display unit 71 selectively displays a three-dimensional image on which a three-dimensional contour plane is superimposed, and a two-dimensional sectional image on which a two-dimensional contour line is superimposed.

The ROI/measurement value display unit 73 creates a graph or a table on the basis of the measurement values calculated by the ROI/measurement value calculator 45 and displays it together with an image group displayed on the image display unit 71.

The controller 8 is connected to each constituent element of the ultrasonic signal generator 2, the ultrasonic image generator 3, the operating unit 4, the storage unit 5, the setting unit 6 and the display unit 7, and collectively controls the constituent elements to function. The specific hardware of the controller 8 is CPU of a computer system.

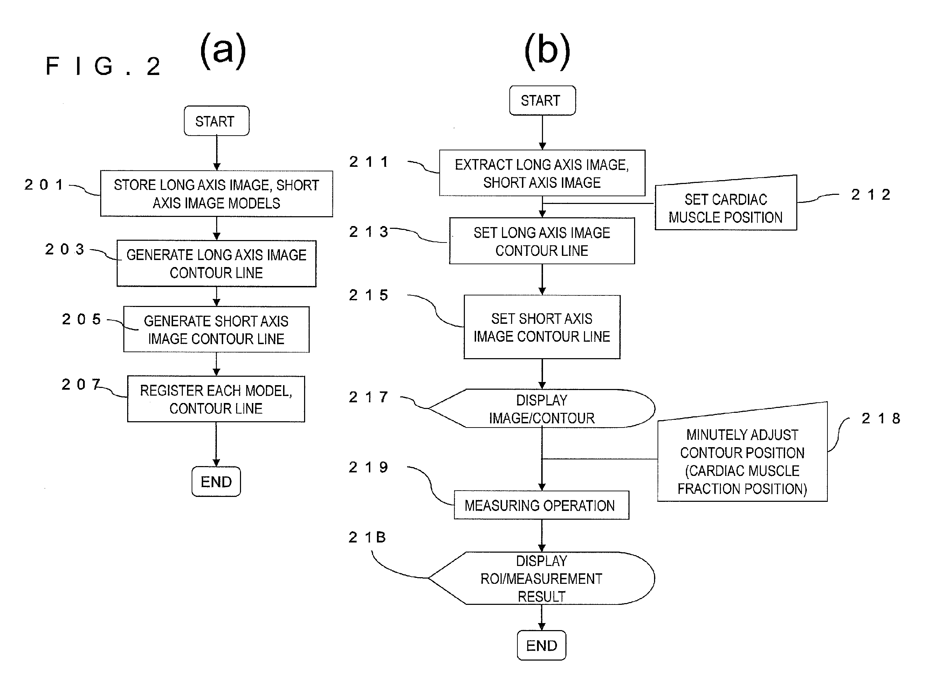

Next, an operation example of this embodiment will be described with reference to Figs. 2, 3 and 4.
Fig. 2 is a flowchart showing the measurement processing of the ultrasonic image diagnosis device according to the embodiment 1 of the present invention.

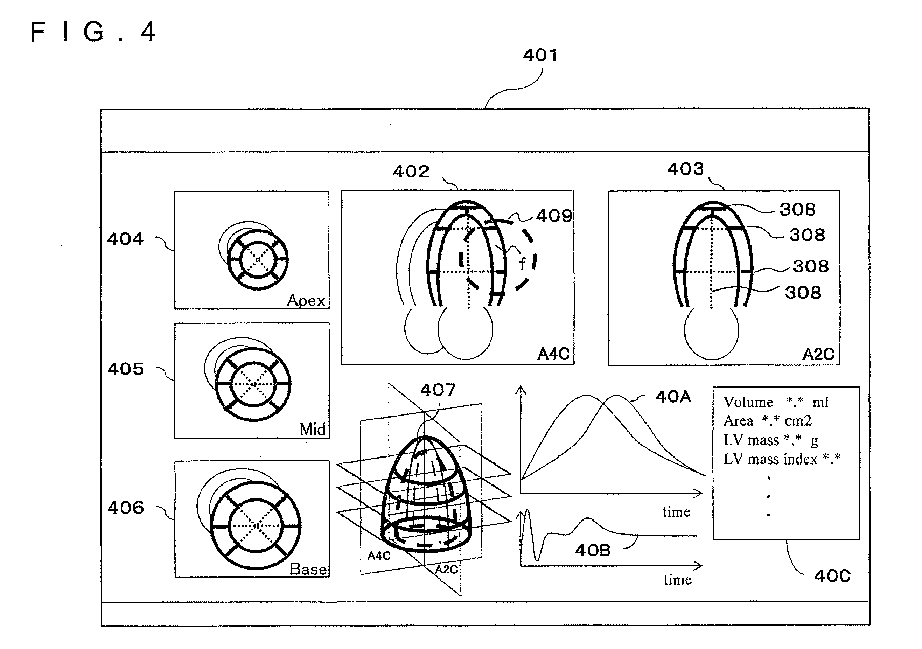

Fig. 3 is a diagram showing an example of the setting of the contour line of Fig. 2. Fig. 4 is a display example of the measurement processing of the ultrasonic image diagnosis device according to the embodiment 1 of the present invention.

Fig. 2(a) is a flowchart showing the process from creation of long axis image and short axis image models to registration of each model, contour lines into the data base portion 53 (referred to as "data base portion registering process"). Fig. 2 (b) is a flowchart showing the process from extraction of a long axis image and a short axis image to display of a measurement result displayed on the ROI/measurement value displaying unit 73 (referred to as "ROI automatic setting process").

The data base portion registering process is executed according to the following procedure shown in Fig. 2 (a) (step 201).
The controller 8 stores models representing the shapes of long axis images such as the image 301b of A2C and the image 301a of A4C and short axis images orthogonal to the long axis images into the data base portion 53. Apex (cardiac apex partial image of short axis image), Mid (papillary muscle partial image of short axis image) and Base (base-of-heart partial image of short axis image) are considered as examples of the short axis image. It is assumed that Apex, Mid, Base are provided to levels 309a, 309b, 309c in the left ventricle at different positions in the long axis direction.

### (Step 203)

The controller 8 generates the contour line of the long axis image on the basis of the ASE fractionating method, and stores it into the storage unit 5. The ASE fractionating method mainly shows that the cardiac muscle of the left ventricle is fractionated. The cardiac muscle exists between the epicardium and the endocardium represented by the heavy lines 302 and 303. According to the 17 fractionating method, the left ventricle is divided into seven cardiac muscle regions a to g by fractionating boundaries 308 (represented by dashed lines) as shown in the image 301a at the upper left side of A4C in Fig. 3. That is, the regions a to g become cardiac muscle fractions. Contour points are provided on the contour lines represented by the heavy lines 302 and 303. The image of A2C which is in such positional relationship as to be orthogonal to the image of A4C is shown at the right side of the image of A4C. The image of A2C is also divided into the seven regions a to g of the cardiac muscle as the cardiac muscle fractions by the fractionating boundaries 308 (represented by dashed lines). Furthermore, as not shown, the type of a new reference section of the long axis image such as A3C (cardiac apex long axis image) or the like may be defined. As described above, the contour line of the long axis image is set at the boundary portion of the cardiac muscle region as shown in the images 301a and 301b.

### (step 205)

The controller 8 generates the contour line of the short axis image on the basis of the ASE fractionating method, and stores it into the storage unit 5. As in the case of the long axis image, the cardiac muscle in the short axis image also exists between the epicardium and the endocardium represented by heavy lines 306 and 307. The short axis image is divided into six cardiac muscle regions by using the positional relationship of the image 301b of A2C orthogonal to the image 301a of A4C of the long axis image. Specifically, it will be described by using a coordinate system in which the positional relationship of the image of A4C and the image of A2C of the long axis images at the lower right side of Fig. 3 is shown. In this coordinate system at the lower right side of Fig. 3, the position of the image of A4C is set to the ordinate axis, and the position of the image of A2C is set to the abscissa axis. The contour lines of the short axis image represented by the heavy lines 306 and 307 intersect to the ordinate axis and the abscissa axis at eight points represented by black circles on the coordinate system at the lower right side of Fig. 3. The cardiac muscle region of the short axis image is divided into six parts by using the relative positional relationship to these eight points.

The short axis image contour lines are set at the boundary portions of the cardiac muscle region as represented by the heavy lines 306 and 307.

### (step 207)

The controller 8 registers the models of the long axis image and the short axis image created in step 201, the contour lines of the long axis image created in step 203 and the contour lines of the short axis image created in step 205 as contour models 304 and 305 in association with the data base portion 53.

Next, the ROI automatic setting process is executed according to the following procedure shown in Fig. 2(b).

### (step 211)

The examiner manually operates the measurement condition setting unit 61 to set parameters for acquiring ultrasonic signals in the controller 8. Upon receiving the set parameters, the controller 8 drives the ultrasonic probe 21 to the ultrasonic signal transmitting/receiving unit 23. The periods of the transmission of an ultrasonic wave and the reception of a reflection signal from the examinee in the ultrasonic probe 21, that is, the transmission and reception periods are switched to each other. The ultrasonic probe 21 transmits an ultrasonic wave to a diagnosis site (for example, a heart or the like) of the examinee during the transmission period. The ultrasonic probe 21 receives a reflection signal from the examinee during the reception period. The ultrasonic signal transmitting/receiving unit 23 phases the received reflection signal, and acquires a three-dimensional ultrasonic signal. This step discloses an example in which a medical image is acquired by a medical image acquiring unit. Furthermore, the process of acquiring the three-dimensional ultrasonic signal is an example of the processing of acquiring a medical image by the medical image acquiring unit.

The controller 8 makes the ultrasonic image generator 3 to create a three-dimensional image constructed by voxel data on the basis of the condition set in the setting unit 6 from the three-dimensional ultrasonic signal input from the ultrasonic signal transmitting/receiving unit 23. The three-dimensional image is generated by the three-dimensional image constructing unit for constructing a three-dimensional image containing a motional internal organ region in the medical image. Furthermore, the step of generating the three-dimensional image is a step of constructing the three-dimensional image containing the motional internal organ region in the medical image by the three-dimensional image constructing unit.

The controller 8 makes the two-dimensional section extracting unit 41 execute an operation of extracting the image of A2C and the image of A4C from the three-dimensional image. The sectional image generator generates two-dimensional cross-sectional images serving as reference images such as A2C and A4C from the three-dimensional image. The step of generating the reference images is a step of generating a two-dimensional cross-sectional image serving as a reference image from the three-dimensional image by the sectional image generator.

Apex, Mid, Base of the short axis image are in such a positional relationship as to be orthogonal to the long axis image such as the image of A2C, the image of A4C, etc., and provided at different positions in the long axis direction in the left ventricle. Apex, Mid, Base of the short axis image are extracted from the cardiac apex side on the basis of the positional relationship thereof.

### (step 212)

The examiner manually operates the measurement condition setting unit 61 so that position information for minutely adjusting the position of the cardiac muscle can be set in the controller 8. Upon receiving the set position information, the controller 8 resets the initial position of the contour model of the cardiac muscle. The precision of the extracted contour which is deformed can be enhanced by initially specifying a rough position of the cardiac muscle in an image. The manual operation of the step 212 is not indispensable, and the controller 8 may be made to extract the position of the cardiac muscle by a publicly known image recognition technique.

### (step 213)

The controller 8 makes the two-dimensional contour line extracting unit 43 extract the contour lines of the epicardium and the endocardium on the image of A2C and the image of A4C. As the contour line extracting method may be applied a method using edge detection processing of detecting variation of an image brightness value of a membrane surface, template matching, a contour model or the like. In this case, the method using the contour model will be described. The contour model is defined by representing the shape of the contour of an object to be extracted or the rule of brightness values in a generalized style. The contour of a heart can be extracted while the contour is adaptively deformed in accordance with the shape of a contour model, that is, the shape of an actual heart. Furthermore, with respect to the contour model, a method of creating a contour model by learning past extracted contour data may be used.

The upper right side of Fig. 3 shows examples of the contour model stored in the data base portion 53, and they are the contour model 304 of the long axis image and the contour model 305 of the short axis image. In general, the neighborhood of the epicardium is liable to be buried in artifact or noise, and thus it is difficult to extract the epicardium alone.

On the other hand, with respect to the endocardium, the brightness of the cardiac muscle and the brightness of the cardiac cavity are relatively clear, so that the endocardium is more easily extracted as compared with the epicardium and the extraction precision of the endocardium is higher than that of the epicardium. With respect to the contour models, they are stored as contour models associating the endocardium and the epicardium with each other, whereby the extraction precision of the epicardium is enhanced while the extraction of the contour of the epicardium is complemented by the extraction data of the endocardium.

### (step 215)

The controller 8 makes the two-dimensional contour line extracting unit 43 extract the contour lines of the endocardium and the epicardium on the short axis images. As shown in Fig. 3, the fractionating boundaries 308 and the positions 309a to 309c of the short axis image level (in this case, three stages of Apex, Mid, Base are set as an example) are stored in the contour model together with the contour lines. The contour model is deformed in conformity with the deformation of the left ventricle of the image.

By extracting the contour as described above, the contour on the image 301a of A4C and the contour on the image 301b of A2C are extracted, and simultaneously the positions 309a to 309c of the short axis image level and the fractionating boundaries 308 are also determined. The determined fractionating boundaries 308 serve as criteria for region division to divide the reference image into plural regions, and the reference image of the A4C image is divided into plural regions along the criteria for region division.

It is disclosed from the step 212 to the step 215 that the region dividing unit divides the reference image into plural regions on the basis of the criteria for region division. Furthermore, the processing from the step 212 to the step 215 is an example of the step of dividing the reference image into plural regions on the basis of the criteria for region division.

### (step 217)

The controller 8 displays the long axis images and the short axis images on the display unit 7. Specifically, the long axis images (the image of A2C, the image of A4C) are displayed on a display screen 401 of the ultrasonic diagnosis device of Fig. 4 as being represented by reference numerals 402 and 403 respectively, and the short axis images (the images of Apex, Mid, Base) are displayed on the display screen 401 of the ultrasonic diagnosis device of Fig. 4 as being represented by reference numerals 404, 405 and 406, respectively. Furthermore, the three-dimensional image may be displayed on the display screen 401 as being represented by reference numeral 407.

### (step 218)

The examiner can set the position information for minutely adjusting the contour position or the cardiac muscle fraction position into the controller 8 by manually operating the measurement condition setting unit 61. Upon receiving the set position information, the controller 8 minutely adjusts the contour position or the cardiac muscle fraction position. The manual operation of the step 218 is not indispensable, and execution of this operation may be omitted when it is unnecessary to minutely adjust the contour position or the cardiac muscle fraction position.

### (step 219)

The controller 8 makes the ROI/measurement value calculator 45 perform the measurement of a region defined by the contour plane, the contour line and the fraction position described above. The ROI/measurement value calculator 45 measures the motion of each cardiac muscle fraction and automatically sets ROI on the basis of the a cardiac muscle fraction which makes an extremely speedy or slow motion in comparison with surrounding cardiac muscle fractions. The motion of each cardiac muscle fraction can be measured by a cardiac muscle tracking method described next.

The cardiac muscle tracking method is a method of extracting a feature point appearing on a frame of an image. The ROI/measurement value calculator 45 detects this feature point every frame to chase the movement of the feature point concerned. For example, in the case of a heart, an echo signal has a large difference in intensity (amplitude) between the cardiac muscle tissue and the blood flow portion of the interior of the heart, and thus the position of the endocardium as the boundary between these two portions can be detected as a feature point by setting a threshold value for the echo signal. The displacement amount of the feature point between frames is limited to a width determined by the speed. Therefore, a search range which is limited so that the position of a feature point in some frame is set to the center thereof is provided, and a feature point in the next frame is searched within this search range, whereby the search time can be shortened. The ROI/measurement value calculator 45 tracks the feature point of the examinee tissue and outputs tissue displacement information.

In the ROI/measurement value calculator 45, the tissue displacement information such as the motion speed of the tissue, etc. is calculated by using the inter-frame moving amount every plural cardiac muscle fractions which are sectioned by the fractionating boundaries 308. Furthermore, the ROI/measurement value calculator 45 calculates statistical values such as an average value, a variance value, a median value, etc. from the motion speed every plural cardiac muscle fractions, and peculiar region position information of a cardiac muscle site at which the speed is peculiarly high or low is calculated by using these statistical values as threshold values. The ROI/measurement value calculator 45 automatically sets the region corresponding to the peculiar region information position information as ROI. The region set as ROI is not limited to one, or plural regions may be provided.

The ROI/measurement value calculator 45 can calculate the distance between the endocardium and the epicardium, that is, the thickness of the cardiac muscle because the position of the cardiac muscle is specified by the endocardium and the epicardium. Furthermore, the ROI/measurement value calculator 45 can calculate the weight of the cardiac muscle by subtracting the volume surrounded by the endocardium from the volume surrounded by the epicardium and multiplying the subtraction volume by the well-known specific gravity of the cardiac muscle. Furthermore, the ROI/measurement value calculator 45 can calculate various kinds of measurement values described above at a specific fraction position because the fraction position is set.

Furthermore, the ROI/measurement value calculator 45 is applicable to the measurement on the two-dimensional contour line. Accordingly, detailed diagnosis can be simultaneously performed by checking the three-dimensional measurement while performing the two-dimensional diagnosis which has been hitherto established.

The heart is an internal organ involving a motion, and thus the diagnosis based on the motion information is important. Therefore, the ROI/measurement value calculator 45 can apply a method of calculating a movement amount of a heart by making the contour plane and the contour line follow the motion of the heart. The movement amount may be calculated by using, as a following method, a follow-up operation such as speckle tracking or the like which has been hitherto proposed, whereby the time variation of the measurement value can be measured. For example, indexes such as volume variation, strain, ejection fraction can be derived.

It is disclosed through the respective steps from the step 212 to the step 215 that the region-of-interest setting unit calculates the motion states of the plural regions, specifies at least one region of the plural regions on the basis of the calculated motion states, and the region of the medical image containing the specified region is set as a region of interest.

The process from the step 217 to the step 219 is an example of the process of calculating the motion states of the plural regions, specifying at least one region of the plural regions on the basis of the calculated motion states and setting the region of the medical image containing the specified region as the region of interest by the region-of-interest setting unit.

### (step 21B)

The controller 8 makes the display unit 7 display ROI and the measurement values in conformity with the long axis images and the short axis images.
ROI is represented by reference numeral 409 in Fig. 4. ROI 409 is a region represented by a dashed-line circle containing a cardiac muscle region f. The display example of ROI 409 is represented by a dashed line, however, when the background image is monochromatic, it may be colored or represented by a line segment such as a solid line, a one-dotted chain line or the like in spite of the dashed line. Furthermore, the shape of ROI 409 is not limited to a circle, but it may be a rectangle or a shape which is obtained by extracting the contour of an internal organ or an organ according to another method and making the shape taken along the extracted contour or approximate to the extracted contour.

Furthermore, the measurement values may be displayed as time variations of the volume in a graph display style like reference numeral 40A, or may be displayed as various kinds of numerical values of the volume, the area, the cardiac muscle weight and the cardiac ejection fraction like reference numeral 40C. Furthermore, a biological signal such as an electrocardiographic wave represented by reference numeral 40B or the like may be displayed together with the various kinds of numerical values.

According to the embodiment 1 described above, a three-dimensional image of a motional internal organ is divided into plural regions and, a peculiar divisional region out of plural regions can be set as ROI.

### Embodiment 2

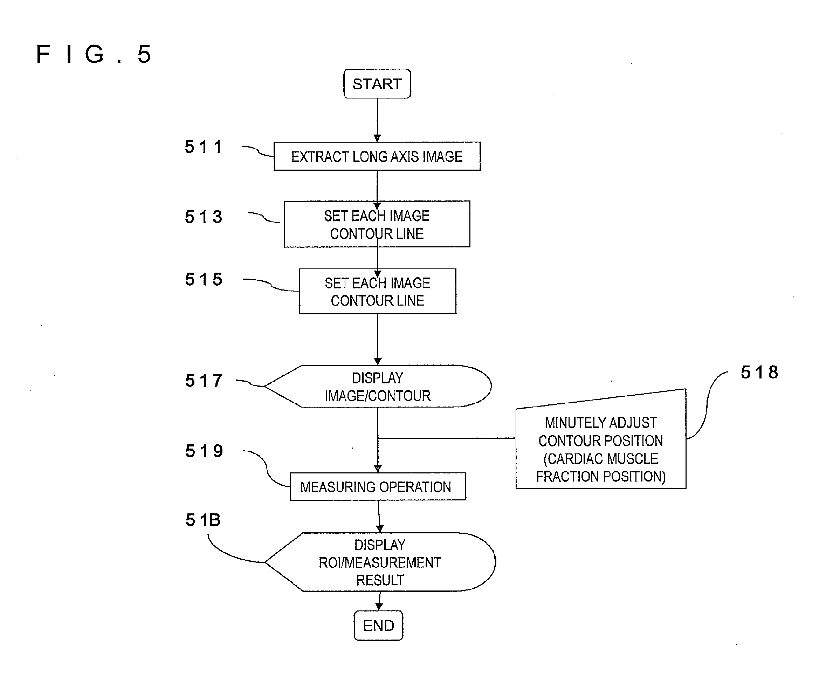

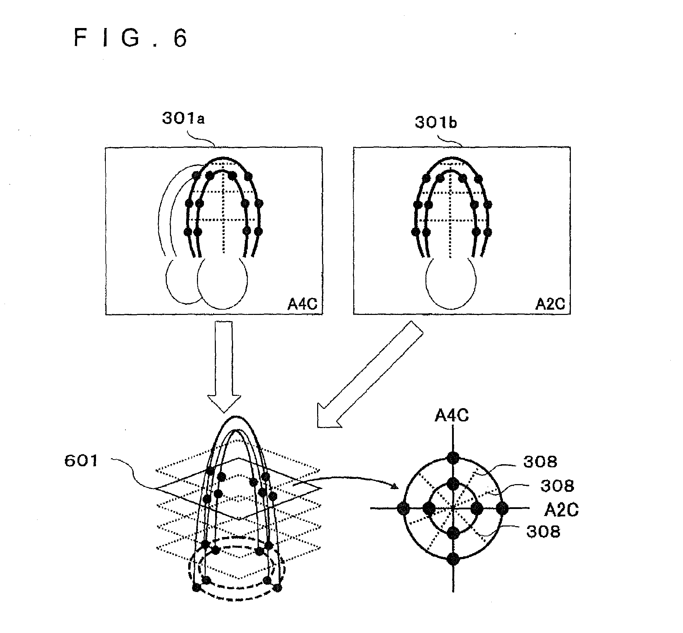

In the embodiment 2, a case where a model stored in the data base portion 53 is not referred to will be described. Fig. 5 is a flowchart showing the measurement processing of an ultrasonic image diagnosis device according to a second embodiment of the present invention. Fig. 6 is a diagram showing an example of the setting of the contour line of Fig. 5.

### (step 511)

The controller 8 extracts the long axis image (the image of A2C and the image of A4C) from the three-dimensional ultrasonic signal by publicly known image recognition. The controller 8 displays the extracted long axis image on the display unit 7. The examiner manually set the endocardiac and epicardiac contours on the image of A2C and the image of A4C for the long axis images displayed on the display unit 7 by using the measurement condition setting unit 61. Furthermore, the examiner further sets the fractionating boundaries of the cardiac muscle fractions (reference numeral 308 of Fig. 4 of the embodiment 1) by using the measurement condition setting unit 61.

### (step 513)

The examiner sets the positions of the short axis images (Apex, Mid, Base) by using the measurement condition setting unit 61 for the long axis images displayed on the display unit 7. The controller 8 displays the short axis images at the set positions on the display unit 7. Subsequently, with respect to the short axis images displayed on the display unit 7, the examiner extracts the endocardiac and epicardiac contours on each short axis image by using the measurement condition setting unit 61. The endocardiac and epicardiac contours of the image of A2C and the image of A4C in step 511 intersect to a short axis section 601 at eight intersecting points as shown at the lower left side of Fig. 6. The examiner sets the contour points for the short axis image displayed on the display unit 7 by using the measurement condition setting unit 61 so as to pass through eight points. Furthermore, the fractionating boundaries 308 of the cardiac muscle fractions are also set on the short axis image as shown at the lower right side of Fig. 6.

### (step 515) - (step 51B)

The above steps are the same as the step 215 to the step 51B described with reference to the embodiment 1, and thus the description is omitted.
With respect to the step 511 or the step 513, a model stored in the data base portion 53 may be referred to for any one of the steps 511 and 513.

According to the embodiment 2 described above, the three-dimensional image of the motional internal organ is divided into plural regions, and a peculiar divisional region out of the plural region can be set as ROI. Furthermore, the examiner can select whether he/she refers to the data base portion 53 or not, and thus the degree of freedom of operability can be increased for the examiner.

### Embodiment 3

In the embodiment 1, the long axis images or the short axis images are orthogonal to one another.
However, it is unnecessary that the long axis images or the short axis images are in orthogonal positional relationship. If they have special angular relationship, the type of the reference section can be freely determined irrespective of "orthogonal" or "non-orthogonal". The different point between the embodiment 1 and the embodiment 3 resides in only the positional relationship of "orthogonal" or "non-orthogonal", and thus only the difference in positional relationship will be described.

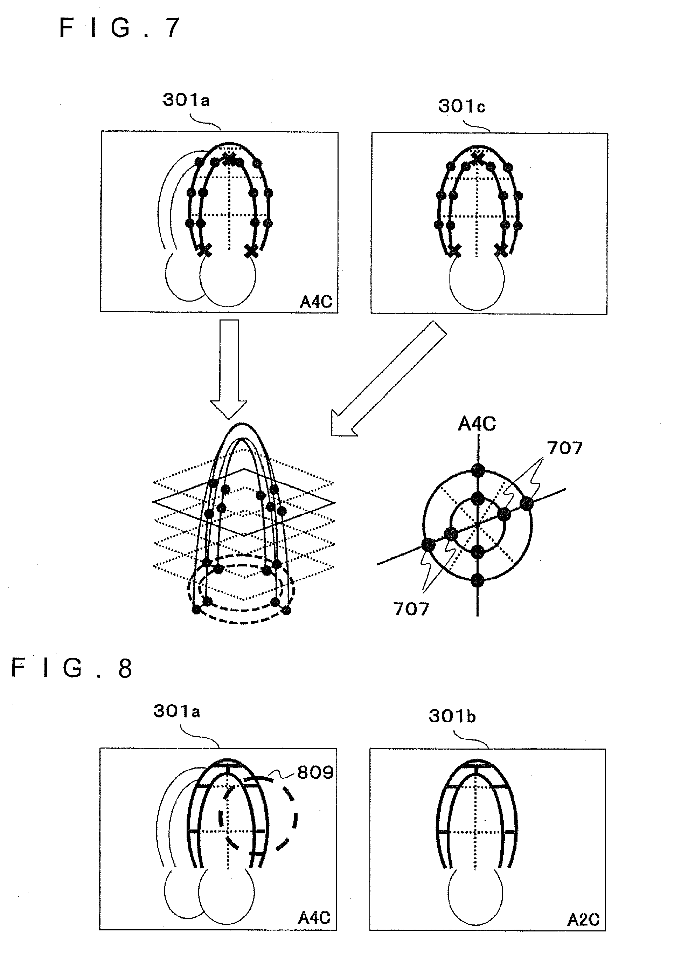

Fig. 7 is a diagram showing an example of the setting of the contour line of the embodiment 3 according to the present invention.
For example, when a three-dimensional contour plane is cut by a long axis section which obliquely intersects to A4C as shown at the lower left side of Fig. 7, an oblique coordinate axis as shown at the lower right side of Fig. 7 is acquired. When the relative positional relationship of intersection points 707 between the oblique coordinate axis and the short axis image contour line and the dividing boundaries is stored in advance, the cardiac muscle region of the short axis image can be divided by using the relative positional relationship.

According to the embodiment 3 described above, the three-dimensional image of the motional internal organ can be divided into plural regions, and a peculiar divisional region out of the plural regions can be set as ROI.

### Embodiment 4

In the embodiment 1, the two long axis images are displayed.
However, it is unnecessary to display two long axis images, and a short axis image can be set insofar as at least one long axis image is displayed. The different point between the embodiment 1 and the embodiment 4 resides in that two long axis images are displayed or one long axis image is displayed.

Fig. 8 shows a display example of the measurement processing of an ultrasonic image diagnosis device according to the embodiment 4 of the present invention.
In Fig. 8, for example when only the image of A2C is manually indicated, only the image of A2C is displayed on the screen, and the image of A4C may not be displayed. Fig. 8 shows an example in which ROI 809 is displayed in the image of A4C as an acquisition result of ROI.

According to the embodiment 4 described above, the three-dimensional image of the motional internal organ can be divided into plural regions, and a peculiar divisional region out of the plural regions can be set as ROI.

### Embodiment 5

The example in which two long axis images are displayed is described with respect to the embodiment 1, and the example in which one long axis image is displayed is described with respect to the embodiment 4.

However, it is not necessary to display any long axis image, and a short axis image can be automatically set insofar as a geometrical setting is preset like the left ventricle is equally divided into four parts as in the case of the embodiment 1. The different point between the embodiment 1 and the embodiment 5 resides in that two long axis images are displayed or no long axis image is displayed.

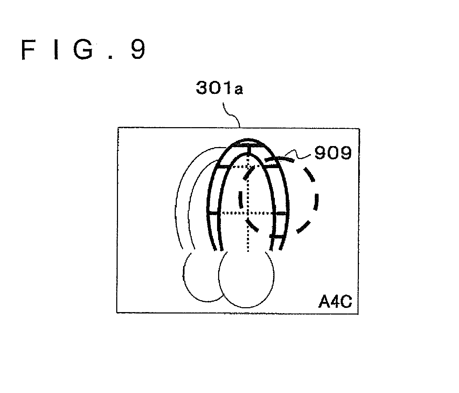

Fig. 9 shows a display example of the measurement processing of an ultrasonic image diagnosis device according to the embodiment 5 of the present invention.
In Fig. 9, for example, a button such as "ROI automatic setting" is prepared in the operating unit 6, and the examiner operates the button of "ROI automatic setting". An example in which ROI 909 is displayed in the image of A4C as an acquiring result of ROI is shown in Fig. 9.

According to the embodiment 5 described above, the three-dimensional image of the motional internal organ can be divided into plural regions, and a peculiar divisional region out of the plural regions can be set as ROI.

As described above, the respective embodiments are described by using the heart as an example of the motional internal organ. However, it is assumed that the motional internal organ contains an internal organ which does not move by itself, but moves in connection with a motion of a motional internal organ, or an internal organ or an organ which moves in connection with a breathing motion.

### Industrial Applicability

The present invention is applicable to various kinds of medical image diagnosis devices such as an ultrasonic diagnosis device, an X-ray CT apparatus, an MRI apparatus, etc. Furthermore, the present invention is also applicable to information equipment which can perform image processing on images obtained from medical image diagnosis devices such as a computer, various kinds of portable terminals, etc.

### Description of Reference Numerals

1 ultrasonic diagnosis device, 2 ultrasonic signal generator, 3 ultrasonic image generator, 4 operating unit, 5 storage unit, 6 setting unit, 7 display unit, 8 controller

## Claims

1. A medical image diagnosis device, **characterized by** comprising:
a medical image acquiring unit that acquires a medical image;
a three-dimensional image constructing unit that constructs a three-dimensional image containing a motional internal organ region in the medical image;
a sectional image generator that generates a two-dimensional sectional image serving as a reference image from the three-dimensional image;
a region dividing unit that divides the reference image into a plurality of regions on the basis of a criterion for region division; and
a region-of-interest setting unit that calculates motion states of the plurality of regions, specifies at least one region of the plurality of regions on the basis of the calculated motion states, and sets a region in the medical image including the specified region as a region of interest.

2. The medical image diagnosis device according to claim 1, wherein the sectional image generator generates a plurality of two-dimensional sectional images, and at least two images of the plurality of two-dimensional sectional images are in orthogonal positional relationship.

3. The medical image diagnosis device according to claim 1, further comprising a data base portion in which a comparison model for generating the two-dimensional sectional images is registered, wherein the sectional image generator generates a two-dimensional sectional image by referring to the comparison model registered in the data base.

4. The medical image diagnosis device according to claim 1, further comprising a data base portion in which a comparison model for the division into the plurality of regions is registered, wherein the region dividing unit divides into the plurality of regions by referring to the comparison model registered in the data base.

5. The medical image diagnosis device according to claim 1, further comprising a setting unit that inputs position information for an image displayed on the display unit to generate the two-dimensional sectional images, and the sectional image generator generates the two-dimensional sectional images on the basis of the position information input by the setting unit.

6. The medical image diagnosis device according to claim 1, further comprising a setting unit that inputs position information for an image displayed on the display unit for the division into the plurality of regions, wherein the region dividing unit divides into the plurality of regions on the basis of the position information input by the setting unit.

7. The medical image diagnosis device according to claim 1, wherein the sectional image generator generates a plurality of two-dimensional sectional images, and at least two images of the plurality of two-dimensional sectional images are in such positional relationship as to have a specific angle.

8. The medical image diagnosis device according to claim 1, wherein the sectional image generator generates at least one two-dimensional sectional image, and the region dividing unit divides the two-dimensional sectional image into a plurality of regions on the basis of a criterion for region division.

9. The medical image diagnosis device according to claim 1, further comprising an initiating unit that initiates a series of constituent units from the three-dimensional image constructing unit till the region-of-interest automatic setting unit, wherein the three-dimensional image constructing unit, the sectional image generator, the region dividing unit and the region-of-interest automatic setting unit are initiated by the initiating unit.

10. The medical image diagnosis device according to claim 1, wherein the region-of-interest setting unit calculates a statistical value by using a motion state calculated every plural regions, and specifies a region by using the statistical value as a threshold value.

11. The medical image diagnosis device according to claim 1, wherein the sectional image generator generates the two-dimensional sectional image by using a contour model having endocardium and epicardium.

12. A region-of-interest setting method for a medical image diagnosis device, **characterized by** comprising:
a first step of acquiring a medical image by a medical image acquiring unit;
a second step of constructing a three-dimensional image containing a motional internal organ region in the medical image by a three-dimensional image constructing unit;
a third step of generating a two-dimensional sectional image serving as a reference image from the three-dimensional image by a sectional image generator;
a fourth step of dividing the reference image into a plurality of regions on the basis of a criterion for region division by a region diving unit; and
a fifth step of calculating motion states of the plurality of regions, specifying at least one region out of the plurality of regions on the basis of the calculated motion states and setting a region in the medical image including the specified region as a region of interest by a region-of-interest setting unit.

13. The region-of-interest setting method for the medical image diagnosis device according to claim 12, wherein the third step generates a plurality of two-dimensional sectional images by the sectional image generator, and at least two images of the plurality of two-dimensional sectional images are in such positional relationship as to be orthogonal to each other.

14. The region-of-interest setting method for the medical image diagnosis device according to claim 12, further comprising a sixth step of inputting position information for an image displayed on the display unit to generate the two-dimensional sectional image by a setting unit, and the third step generates the two-dimensional sectional image on the basis of the position information input through the setting unit by the sectional image generator.

15. The region-of-interest setting method for the medical image diagnosis device according to claim 12, further comprising a seventh step of inputting position information for an image displayed on the display unit to divide into the plurality of regions by a setting unit, and the third step divides into the plurality of regions on the basis of the position information input through the setting unit by the sectional image generator.

16. The region-of-interest setting method for the medical image diagnosis device according to claim 12, wherein the third step generates a plurality of two-dimensional sectional images by the sectional image generator, and at least two images of the plurality of two-dimensional sectional images are in such positional relationship as to have a specific angle.

17. The region-of-interest setting method for the medical image diagnosis device according to claim 12, wherein the third step generates at least one two-dimensional sectional image by the sectional image generator, and the fourth step divides the one two-dimensional sectional image into a plurality of regions on the basis of a criterion for region division by the region dividing unit.

18. The region-of-interest setting method for the medical image diagnosis device according to claim 12, further comprising an eighth step of initiating a series of constituent units from the three-dimensional image constructing unit till the region-of-interest automatic setting unit by an initiating unit, wherein the three-dimensional image constructing unit, the sectional image generator, the region dividing unit and the region-of-interest automatic setting unit are initiated by the initiating unit in the eighth step.
